(19) Europäisches Patentamt
European Patent Office
Office européen des brevets

(11) **EP 3 440 185 B1**

(12) **FASCICULE DE BREVET EUROPEEN**

(45) Date de publication et mention de la délivrance du brevet:
**03.05.2023   Bulletin 2023/18**

(21) Numéro de dépôt: **17721765.0**

(22) Date de dépôt: **03.04.2017**

(51) Classification Internationale des Brevets (IPC):
**C12M 1/00** *(2006.01)*      **C12M 1/42** *(2006.01)*

(52) Classification Coopérative des Brevets (CPC):
**C12M 21/02; C12M 35/02; C12M 41/06**

(86) Numéro de dépôt international:
**PCT/FR2017/050765**

(87) Numéro de publication internationale:
**WO 2017/174907 (12.10.2017 Gazette 2017/41)**

(54) **BIORÉACTEUR SÉLECTIF POUR MICROALGUES**

**SELEKTIVER BIOREAKTOR FÜR MIKROALGEN**

**SELECTIVE BIOREACTOR FOR MICROALGAE**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **04.04.2016   FR 1652933**

(43) Date de publication de la demande:
**13.02.2019   Bulletin 2019/07**

(73) Titulaires:
• **INRIA - Institut National de Recherche en Informatique et en Automatique**
**78150 Le Chesnay (FR)**
• **Centre National de la Recherche Scientifique CNRS**
**75016 Paris (FR)**

(72) Inventeurs:
• **MAIRET, Francis**
**06560 Valbonne (FR)**
• **BERNARD, Olivier**
**06510 Carros (FR)**
• **BONNEFOND, Hubert**
**06000 Nice (FR)**
• **SCIANDRA, Antoine**
**06230 Villefranche sur mer (FR)**
• **PRUVOST, Eric**
**06100 Nice (FR)**

(74) Mandataire: **Vidon Brevets & Stratégie**
**16B, rue de Jouanet**
**BP 90333**
**35703 Rennes Cedex 7 (FR)**

(56) Documents cités:
KR-A-020040 059 182      US-A- 5 882 849
US-A1- 2007 231 886      US-A1- 2014 356 902

**Description**

La présente invention se rapporte à un bioréacteur pour sélectionner des souches de microalgues. L'invention se rapporte également à un procédé de sélection de souches de microalgues.

Il existe des micro-organismes photosynthétiques procaryotes et eucaryotes, regroupés communément sous le terme de microalgues. Les micro-organismes photosynthétiques procaryotes sont représentés par les cyanobactéries (parfois appelées « algues bleu-vert »). Les micro-organismes photosynthétiques eucaryotes sont représentés par une multitude de classes parmi lesquelles on peut citer les chlorophycées, les diatomées, les chrysophycées, les coccolithophycées, les euglénophycées et les rhodopycées. D'une manière générale, la taille d'une cellule de microalgue est comprise entre 1 $\mu$m et 100 $\mu$m.

On estime aujourd'hui qu'il existe plus d'un million d'espèces de microalgues dont quelques dizaines de milliers d'espèces sont référencées. Les microalgues sont ubiquistes et on les trouve aussi bien dans les eaux douces et que dans les eaux saumâtres et marines.

La production de microalgues est un secteur qui prend de plus en plus d'ampleur. En effet, les microalgues synthétisent de nombreux produits de natures différentes parmi lesquels on peut citer les protéines, les antioxydants, les pigments, les acides gras polyinsaturés à longues chaînes DHA (acide docosahexaénoïque) et EPA (acide eicosapentaénoïque).

Ainsi, les microalgues trouvent une application dans plusieurs domaines technologiques et notamment dans l'industrie cosmétique, l'industrie pharmaceutique, l'aquaculture, l'industrie des alicaments ou des compléments alimentaires.

De plus, les microalgues sont utilisées dans la production de bioénergie. Les microalgues ont une capacité à capter l'énergie lumineuse pour fixer et métaboliser le carbone inorganique à partir du dioxyde de carbone ($CO_2$) dans des molécules énergétiques. Le couplage des microalgues avec le $CO_2$ et le fait que les microalgues sont souvent riches en sucres ou en huiles ont pour conséquence que les microalgues présentent un grand intérêt dans la production de biocarburants. Ce couplage est aussi à l'origine de capacités épuratoires que présentent les microalgues.

Les microalgues sont des espèces photosynthétiques. Les cellules de microalgues ont besoin de la lumière pour proliférer. Les microalgues peuvent être cultivées en utilisant de la lumière naturelle (lumière solaire) ou de la lumière artificielle. Il existe des systèmes de culture ouverts de type bassin de culture (aussi appelé bassin « raceway») et des systèmes de culture fermés comme les bioréacteurs d'alimentation par batch, les bioréacteurs d'alimentation par fed-batch ou les bioréacteurs d'alimentation continu.

D'une manière générale, tous les systèmes de culture présentent une cuve destinée à recevoir un milieu de culture qui comprend des nutriments. Les microalgues sont dispersées dans ce milieu de culture et reçoivent de la lumière. Les bioréacteurs d'alimentation en continu ont l'avantage de présenter une entrée et une sortie de milieu de culture associées à un contrôleur. Le contrôleur permet de piloter la cuve en continue à une concentration choisie de microalgues dans le milieu de culture durant un temps de travail.

Dans les systèmes de cultures ouverts ou fermés, la lumière est majoritairement absorbée par les cellules proches de la source lumineuse. Lorsque la densité de microalgues est élevée, une grande partie de la lumière ne parvient pas à pénétrer en profondeur de la cuve. En conséquence, les microalgues en profondeur des cuves se retrouvent dans l'obscurité et ne peuvent pas proliférer correctement.

Les systèmes de cultures présentent un problème de surexposition lumineuse des cellules proches de la source lumineuse et un problème de sous-exposition des cellules situées en profondeur du milieu de culture. Ce gradient de lumière au sein des systèmes de culture limite la production de microalgues.

L'état de la technique propose une approche qui consiste à modifier génétiquement les microalgues. Il s'agit de réduire la taille ou le nombre des antennes collectrices (molécules de chlorophylle) et/ou de modifier les ratios entre les différents pigments (notamment chlorophylle a, chlorophylle b, chlorophylle c, caroténoïdes et autres pigments) des microalgues pour les rendre plus transparentes.

Le document WO 2014/089533 divulgue des microalgues mutantes présentant des antennes collectrices réduites.

Dans cette approche, chaque cellule de microalgue capte une quantité moindre de lumière ce qui permet à la lumière de pénétrer en profondeur des cuves. Les cellules localisées dans les zones les plus éloignées de la source lumineuse sont donc moins ombragées. Toutefois, cette approche requiert des méthodes complexes et coûteuses de génie génétique ou de mutation-sélection telles que la mutagenèse chimique ou le traitement par ultra violet ou irradiation gamma. Par ailleurs, les systèmes de cultures ne comprennent qu'un seul type de mutant : il s'agit de monocultures de microalgues. Les monocultures sont moins robustes, notamment face à des conditions d'exploitation industrielle. L'état de la technique propose également une approche qui consiste à sélectionner des souches de microalgues riches en composants utilisés dans la production de biocarburants.

Le document WO 2013/012329 divulgue un procédé de sélection de microalgues présentant une capacité de stockage accrue en composants utiles pour la production de biocarburants.

Le document US 2007/0231886 divulgue une installation de réacteur et un procédé pour la culture de microorganismes phototropiques. Le document US 5,882,849 divulgue un procédé pour contrôler la croissance de Hoemotococcus spp. Le document US 2014/0356902 divulgue un procédé pour découvrir des souches d'algues ayant des pigments réduits pour atteindre une efficacité photosynthétique plus élevée. Le document KR 10200 4005 9182 divulgue un procédé pour améliorer la productivité cellulaire en réduisant les pigments photosynthétiques de cellules photosynthétiques.

Dans cette approche, une culture de différentes souches de microalgues est soumise à un stress nutritionnel. Seules les souches capables de stocker une forte concentration en nutriments survivent à ce stress. Les souches sont récoltées et cultivées dans les systèmes de cultures décrits plus hauts. Les problèmes liés au gradient de lumière persistent.

La présente invention vient améliorer la situation.

À cet effet, l'invention vient introduire un bioréacteur comportant une source de lumière, un capteur de lumière en regard de ladite source de lumière et une cuve disposée entre la source de lumière et le capteur de lumière destiné à recevoir un milieu de culture comprenant une culture cellulaire de micro-organismes photosynthétiques, un contrôleur relié au capteur de lumière pour piloter la cuve à une concentration de culture cellulaire choisie dans le milieu de culture durant un temps de travail, ladite source de lumière étant capable d'émettre une lumière incidente d'une intensité lumineuse d'entrée en direction de la cuve, et le capteur de lumière étant capable de mesurer une intensité lumineuse en sortie et de transmettre des données relatives à cette intensité au contrôleur pour le pilotage de la cuve, et une commande de la source de lumière agencée pour régler, durant un temps inférieur ou égal audit temps de travail, l'intensité lumineuse d'entrée à une valeur de consigne permettant d'induire un stress cellulaire chez certains au moins desdits micro-organismes photosynthétiques telle que défini dans les revendications annexées.

La valeur de consigne peut être supérieure ou égale à 500 $\mu$mol quanta m$^{-2}$ s$^{-1}$. Elle est de préférence supérieure ou égale à 800 $\mu$mol quanta m$^{-2}$ s$^{-1}$. La concentration de culture cellulaire choisie ($x_i$) dans le milieu de culture peut être comprise entre 0,001 g/l à 10 g/L.

Selon un mode de réalisation, la concentration de culture cellulaire choisie ($x_i$) dans le milieu de culture est comprise entre 0,001 g/l à 0,1 g/L, de préférence entre 0,05 g/l à 0,1 g/L.

Selon l'invention, la commande est agencée pour régler ladite lumière à une intensité lumineuse d'entrée ($I_{in}$) à une valeur de repos inférieure à la valeur de consigne, et pour commuter entre la valeur de repos et la valeur de consigne durant le temps de travail. La commande est en outre agencée agencée pour fixer un premier temps d'irradiation pour lequel la lumière est réglée à l'intensité lumineuse d'entrée ($I_{in}$) à la valeur de repos et un deuxième temps d'irradiation pour lequel la lumière est réglée à la valeur de consigne, le premier temps d'irradiation étant supérieur au deuxième temps d'irradiation, et pour appliquer une succession des premiers et deuxième temps d'irradiations. Les premiers et deuxième temps d'irradiations sont fixés de sorte que la valeur moyenne de l'intensité lumineuse d'entrée ($I_{in}$) durant le temps de travail calculée à partir de la valeur de repos et la valeur de consigne soit inférieure ou égale à un tiers de ladite valeur de consigne. Dans un mode de réalisation, le premier temps d'irradiation est au moins trois fois supérieur au deuxième temps d'irradiation. La commande peut être agencé pour fixer une pluralité de premiers et deuxièmes temps d'irradiation durant lesquels la lumière est réglée à une intensité lumineuse d'entrée ($I_{in}$) à la valeur de repos lors de chaque premier temps d'irradiation et la lumière est réglée à une intensité lumineuse d'entrée ($I_{in}$) à la valeur consigne lors de chaque deuxième temps d'irradiation. La commande peut être agencé pour fixer la valeur de consigne à chaque deuxième temps d'irradiation.

Dans un autre mode de réalisation la concentration de culture cellulaire choisie ($x_i$) dans le milieu de culture est comprise entre 0,1 g/l à 10 g/L, de préférence entre 1 g/l à 10 g/L. Dans ce mode, la commande peut être agencée pour maintenir l'intensité lumineuse d'entrée ($I_{in}$) à la valeur de consigne de sorte que l'intensité lumineuse en sortie ($I_{out}$) soit sensiblement constante durant un temps inférieur ou égale au temps de travail.

L'invention vise également un procédé de sélection de micro-organismes photosynthétiques, comprenant les étapes suivantes :

1. Mettre à disposition un bioréacteur comportant une source de lumière, et une cuve disposée en regard de la source de lumière destinée à recevoir un milieu de culture comprenant une culture cellulaire de micro-organismes photosynthétiques, ladite source de lumière étant capable d'émettre une lumière d'une intensité lumineuse d'entrée $I_{in}$ en direction de la cuve,

2. Emplir la cuve d'un milieu de culture ;

3. Inoculer le milieu de culture avec une culture cellulaire constituée de micro-organismes photosynthétiques ;

4. Piloter la cuve durant un temps de travail à une concentration de culture cellulaire $x_i$ choisie dans le milieu de culture ;

5. Régler l'intensité lumineuse d'entrée $I_{in}$, à une valeur de consigne permettant d'induire un stress cellulaire chez certains au moins desdits micro-organismes photosynthétiques ;

6. Maintenir l'intensité lumineuse d'entrée $I_{in}$, à la valeur de consigne durant un temps inférieur ou égal audit temps de travail de manière à provoquer une dégradation cellulaire d'au moins une partie desdits micro-organismes photosynthétiques et ainsi sélectionner les micro-organismes photosynthétiques présentant une résistance accrue à la photoinhibition ; et

7. Récolter les micro-organismes photosynthétiques présentant une résistance accrue à la photoinhibition dudit milieu de culture tel que défini dans les revendications annexées.

La récolte à l'étape 7. peut être réalisée lorsque le milieu de culture comporte une culture cellulaire de micro-organismes photosynthétiques constituée à plus de 75%, préférentiellement à plus de 90%, encore plus préférentiellement sensiblement à 100% de micro-organismes présentant une résistance accrue à la photoinhibition.

Dans un mode de réalisation, le procédé met en oeuvre une répétition successive des étapes 5. et 6. jusqu'à l'obtention d'une culture cellulaire de micro-organismes photosynthétiques constituée de micro-organismes présentant une résistance accrue à la photoinhibition.

Selon l'invention, l'étappe 6. du procédé de l'invention comprend les sous-étapes suivantes:

6a. Régler la lumière à une intensité lumineuse d'entrée ($I_{in}$) à une valeur de repos inférieure à la valeur de consigne durant un temps inférieur audit temps de travail ;

6b. Maintenir l'intensité lumineuse d'entrée ($I_{in}$,) à la valeur de repos durant un temps inférieur audit temps de travail ; et

6c. Commuter successivement entre la valeur de repos et la valeur de consigne durant le temps de travail ainsi que les sous-étapes définis dans les revendications.

Le procédé de l'invention peut en outre comprendre les étapes suivantes :

8. Inoculer un milieu de culture avec les micro-organismes photosynthétiques récoltés à l'étape 7. dans un bioréacteur ; et

9. Piloter le bioréacteur de l'étape 8. pour une production de biomasse constituée desdits micro-organismes photosynthétiques.

Le bioréacteur mis en oeuvre dans le procédé peut en outre comprendre un capteur de lumière disposé à l'opposé de la source de lumière par rapport à la cuve, le capteur de lumière étant capable de mesurer une intensité lumineuse de sortie ($I_{out}$). Dans ce mode de réalisation, le procédé comprend en outre l'étape 4a. de mesurer l'intensité lumineuse en sortie ($I_{out}$). Dans ce mode de réalisation l'intensité lumineuse d'entrée ($I_{in}$) à l'étape 5. est réglée en fonction de l'intensité lumineuse en sortie ($I_{out}$) mesurée à l'étape 4a.

D'autres avantages et caractéristiques de l'invention apparaîtront à la lecture de la description détaillée ci-après et sur les dessins annexés sur lesquels :

- la figure 1 montre un schéma de production de microalgues dans un système de culture ;
- la figure 2 montre un schéma de principe du bioréacteur de l'invention durant un temps de travail ;
- la figure 3 montre un schéma de principe du bioréacteur de la figure 2 en fin du temps de travail ;
- la figure 4 montre un diagramme sur lequel figure l'opacité d'une souche sauvage de microalgue et l'opacité d'une souche sélectionnée par le bioréacteur de l'invention ; et
- la figure 5 montre la production de biomasse de la souche sauvage et la souche sélectionnée de la figure 4.

Les dessins et la description ci-après contiennent, pour l'essentiel, des éléments de caractère certain. Ils font partie intégrante de la description, et pourront donc non seulement servir à mieux faire comprendre la présente invention, mais aussi contribuer à sa définition, le cas échéant.

Pour une bonne production de microalgues (ou biomasse de microalgues) il convient de les cultiver dans un milieu de culture riche en nutriments (azote, phosphore, souffre, oligo-éléments, vitamines) et d'apporter suffisamment de lumière. La présence des nutriments est nécessaire pour permettre aux microalgues de convertir l'énergie lumineuse en métabolisant le $CO_2$. Cette conversion a pour conséquence la production d'oxygène et l'augmentation en biomasse par la prolifération des microalgues (multiplication par division cellulaire).

Classiquement on utilise une source de lumière capable d'émettre une lumière à une longueur d'onde qui est fortement absorbée par les microalgues, afin d'obtenir un taux de croissance élevé.

Les publications Light requirements in microalgal photobioreactors: on overview of biophotonic aspects - Carvalho et al., Appl Microbiology and Biotechnology, 2011, vol. 89, no. 5: 1275-1288 et Light emitting diodes (LEDs) applied to microalgal production - Schulze et al., Trends in Biotechnology, 2014, vol. 32, no. 8: 422-430 décrivent l'utilisation de la lumière dans les systèmes de culture des microalgues pour obtenir une bonne croissance cellulaire.

Dans un bioréacteur, les microalgues peuvent recevoir un excès de photons qui peut entraîner une baisse de l'efficacité photosynthétique. Ceci correspond à une dissipation de l'énergie appelée dissipation non-photochimique (en anglais : non-photochemical quenching). Le phénomène de la dissipation non-photochimique est notamment décrit dans la publication Non-Photochemical Quenching. A Response to Excess Light Energy - Müller et al., Plant Physiol, 2001, vol. 125, no. 4: 1558-1566. De plus, l'excès d'absorption de photons peut provoquer une dégradation de l'appareil photosynthétique qui diminue, voire inhibe, la prolifération des microalgues. Ce phénomène est appelé photoinhibition.

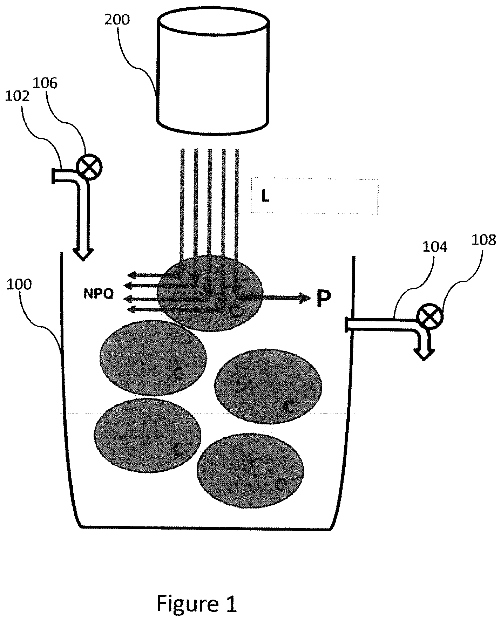

La figure 1 montre un schéma de production de biomasse de microalgues dans un bioréacteur d'alimentation continue.

Le bioréacteur comprend une cuve 100 apte à recevoir un milieu de culture comportant des microalgues. Les microalgues sont dispersées dans le milieu de culture ou sous forme de biofilm. Les microalgues sont constituées de cellules C de micro-organismes photosynthétiques. Le bioréacteur comprend une entrée 102 et une sortie 104 respectivement associées à un dispositif de régulation de débit. Ainsi, l'entrée 102 et la sortie 104 sont respectivement associées à une première vanne (ou pompe) 106 et une deuxième vanne (ou pompe) 108 pour ouvrir et fermer l'entrée 102 et la sortie 104. L'entrée 102 et la première vanne 106 permettent de contrôler l'apport du milieu de culture frais dans la cuve 100. La sortie 104 et la deuxième vanne 108 permettent de contrôler l'évacuation du milieu de culture et, le cas échéant, de certaines au moins des cellules C. L'entrée et la sortie permettent de piloter en continu le bioréacteur pour une production P de biomasse de microalgues.

D'une manière générale, le contrôle de la production de biomasse dans un système de culture à alimentation en continu s'appuie sur la maîtrise de la croissance cellulaire de la culture de microalgues. La concentration x [g/L] de microalgues dans le milieu de culture évolue en fonction du taux de croissance spécifique $\mu(x)$ [h$^{-1}$] et du taux de dilution $D$ [h$^{-1}$] du milieu de culture. Le taux de dilution D est défini par le débit d'entrée (L/h) divisé par le volume (L) du milieu de culture.

La concentration x [g/L] de microalgues dans le milieu de culture évolue au cours du temps. Pour une souche de microalgues donnée cette évolution peut être exprimée par la formule F1 suivante :

$$\dot{x} = \mu\, x - D\, x \qquad\qquad [F1]$$

En conséquence, pour la production de biomasse dans la cuve 100 du bioréacteur (à volume constant):

- Si $\mu(x) > D$ : les cellules se multiplient (par division cellulaire) plus vite qu'elles ne sont évacuées, leur nombre et donc leur concentration (biomasse) va augmenter.
- Si $\mu(x) < D$ : les cellules se multiplient (par division cellulaire) moins vite qu'elles ne sont évacuées, leur nombre et donc leur concentration (biomasse) va diminuer.
- Si $\mu(x) = D$ : le nombre de cellules demeure constant au cours du temps. Le nombre de cellules évacuées avec le milieu de culture de la cuve est égal au nombre de cellules obtenues par leur multiplication dans le milieu de culture au sein de la cuve. La concentration est stable.

Le bioréacteur comprend une source de lumière 200. La source de lumière 200 est apte à émettre une lumière incidente L. La lumière L est typiquement choisie pour couvrir tout le spectre solaire incluant la lumière bleue (de préférence de 430 nm à 470 nm) et rouge (de préférence de 650 nm à 700 nm). Ces plages de longueurs d'ondes permettent un bon taux de croissance des microalgues car elles sont fortement absorbées par celles-ci.

Les phénomènes d'optique, d'absorbance et de métabolisation de photons dans une cuve de bioréacteur sont détaillés dans les ouvrages Microalgal biotechnology: potential and production, C. Posten and C. Walter, de Gruyter, 2012 et Handbook of Microalgal Culture: Applied Phycology and Biotechnology, 2ème Édition, A. Richmond and Q. Hu, Wiley-Blackwell, 2013.

La lumière incidente L éclaire essentiellement les cellules de microalgues proches de la source de lumière 200. Ces microalgues reçoivent la majorité des photons émis par la source de lumière 200. Cela induit le phénomène de dissipation non-photochimique NPQ (en anglais : Non-Photochemical Quenching), et également de la photoinhibition. Les cellules de microalgues situées en profondeur de la cuve 100 ne sont pas éclairées, ou presque pas, ce qui les empêche de proliférer. Ceci s'applique surtout à forte densité cellulaire, c'est-à-dire lorsque la concentration de microalgues dans la cuve 100 est élevée (par exemple entre 1 g/L et 10 g/L).

La production P de biomasse résultante n'est pas satisfaisante. Pour répondre à ce problème, certains systèmes de culture de l'état de la technique utilisent des outils de mélange améliorés favorisant une circulation des microalgues au sein de la cuve. De cette façon, toutes les cellules sont exposées à la lumière.

Toutefois, l'utilisation de ces outils présente un coût énergétique important et les problèmes existants liés à la photoinhibition et au gradient de lumière ne sont pas résolus.

La présente invention propose une approche radicalement différente. Ainsi, au lieu d'essayer d'éviter les effets de la photoinhibition, l'invention propose d'en faire un avantage.

La Demanderesse a découvert non sans surprise que dans un système de culture la photoinhibition permet de sélectionner des microalgues convenants à une exploitation industrielle. Le système peut être piloté en continu ou en semi-continu de type *fed-batch.*

Certaines espèces de microalgues sont capables d'ajuster leur contenu en pigments photorécepteurs en fonction de la lumière reçue. Ce phénomène est appelé photoacclimatation. Les microalgues exposées à une forte intensité lumineuse, s'acclimatent à cette forte intensité lumineuse et deviennent plus translucides. Les microalgues deviennent notamment plus translucides en diminuant la présence de pigments photorécepteurs au sein des cellules. Les cellules de microalgues deviennent ainsi plus résistantes à la photoinhibition. On connaît par exemple l'espèce *Chlorella sorokiniana* qui présente une résistance accrue vis-à-vis de la photoinhibition, cf. Chlorella sorokiniana UTEX 2805, a heat and intense, sunlight-tolerant microalga with potential for removing ammonium from wastewater - Bashan, L. E., Trejo, A., Huss, V. A., Hernandez, J. P., & Bashan, Y., Bioresource Technology, 99(11), 4980-4989, 2008. De manière générale, la résistance à de fortes intensités lumineuse peut provenir de la capacité de certaines espèces à décroître leur contenu pigmentaire. Dans des systèmes de culture, ces microalgues translucides laissent passer une grande partie de la lumière incidente en profondeur de la cuve. Le problème lié au gradient de lumière dans la cuve est restreint, voir résolu.

Toutefois, peu d'espèces à résistance accrue à la photoinhibition sont connues aujourd'hui.

Par ailleurs, lorsque ces espèces de microalgues sont exposées à une faible intensité lumineuse, elles s'acclimatent à cette faible intensité lumineuse et deviennent plus opaques. Ainsi, dans les systèmes de culture à forte densité, par exemple une concentration de microalgues supérieure à 0,1 g/L, l'intensité lumineuse moyenne en profondeur de la cuve est faible. En conséquence, les microalgues perdent le bénéfice de leur transparence acquise lors de l'exposition à une forte intensité lumineuse.

Un objectif de l'invention est donc notamment de générer de nouvelles souches de microalgues qui, même à des lumières faibles (à intensité lumineuse faible), présentent une faible teneur en pigments et/ou qui procèdent à une modification de l'appareil pigmentaire. Des biomasses plus importantes dans les systèmes de cultures peuvent alors être obtenues. D'une manière générale, un stress lumineux à pour conséquence une adaptation des cellules de microalgues. Cette adaptation se traduit notamment par des mutations génétiques au sein des cellules de microalgues. En d'autres termes, les mutations génétiques naturelles induites par le stress lumineux conduisent à des mécanismes d'adaptation génétique. Les mutations génétiques sont susceptibles d'être transmises d'une cellule à ses descendantes, et donc de se transmettre de génération à génération. Une mutation génétique peut, par exemple, résulter en la diminution de la teneur en photorécepteurs (tel que la chlorophylle) dans les cellules de microalgues. Elle peut également conduire à augmenter la vitesse de recyclage des pigments, et donc leur production/dégradation active ou encore à une modification des ratios des différents pigments de la cellule. De cette manière, les microalgues deviennent plus translucides et laissent passer une grande partie de la lumière incidente, ce qui restreint ou résout le problème lié au gradient de lumière dans les cuves de systèmes de culture.

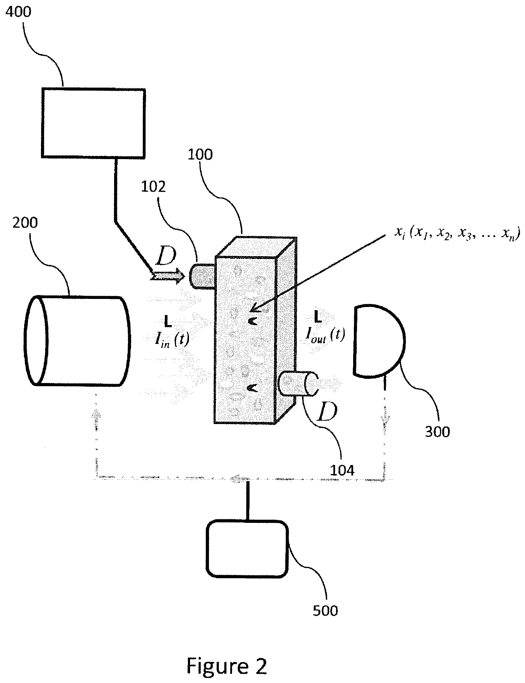

La figure 2 montre un schéma de principe du bioréacteur de l'invention durant un temps de travail.

Le bioréacteur comprend une cuve 100 avec un milieu de culture. Le milieu de culture comprend une dispersion d'une culture cellulaire de micro-organismes photosynthétiques. La culture cellulaire comprend n différentes souches de micro-organismes photosynthétiques (on entend par « différentes souches » des différents génotypes ou phénotypes d'une ou plusieurs espèces de microalgues) de concentrations respectives $x_1, x_2, x_3...x_n$. Les différentes souches de micro-organismes photosynthétiques sont plus ou moins résistantes à la photoinhibition. La cuve 100 présente des moyens de mélange du milieu de culture.

Le bioréacteur comprend en outre une entrée 102 et une sortie 104. L'entrée 102 permet d'apporter du milieu de culture frais dans la cuve 100. La sortie 104 permet d'évacuer du milieu de culture et des microalgues de la cuve 100.

L'entrée 102 et la sortie 104 sont associées à un contrôleur 400. Le contrôleur 400 permet de piloter la cuve 100 en continu pendant un temps de travail. Le temps de travail varie selon les souches de microalgues présentes dans le milieu de culture. Le temps de travail s'étend préférentiellement à minima sur 1 mois (sans limite supérieure de temps) ce qui correspond généralement à au moins 20 générations de microalgues (c'est-à-dire 20 divisions cellulaire successives).Selon l'invention, le temps de travail est supérieur au temps nécessaire pour un cycle cellulaire de microalgues. Dans un mode de réalisation, le temps de travail s'étend de 6 à 12 mois.

Le contrôleur 400 pilote la cuve 100 en continu à un taux de dilution *D.* Pour cela, le contrôleur 400 comprend une ou plusieurs cellules photoélectriques pour mesurer la densité optique de l'ensemble milieu de culture/microalgues au sein de la cuve 100. De cette manière le contrôleur peut maintenir la concentration de culture cellulaire $\Sigma x_i$ dans le milieu de culture entre 0,001 g/L et 10 g/L, de préférence entre 0,05 g/L à 0,1 g/L ou entre 0,1 et 5 g/L, durant le temps de travail et adapter le taux de dilution *D* en apportant et évacuant du milieu de culture. En d'autres termes, la concentration totale constituée des concentrations respectives $x_1, x_2, x_3...x_n$ des différentes souches de microalgues est comprise entre 0,05 g/L à 0,1 g/L durant le temps de travail. Cette concentration permet une bonne diffusion de la lumière au sein de la cuve 100

Le bioréacteur comprend une source de lumière 200 qui émet une lumière incidente L d'une intensité lumineuse d'entrée $I_{in}$ suffisamment élevée pour traverser la cuve 100 emplie du milieu de culture comprenant la dispersion de microalgues. Dans le présent mode de réalisation, la source de lumière 200 est capable d'émettre une intensité lumineuse d'entrée $I_{in}$ qui peut aller jusqu'à 5000 $\mu$mol quanta m$^{-2}$ s$^{-1}$.

Le bioréacteur comprend en outre un capteur de lumière 300 disposé en regard de ladite source de lumière pour mesurer l'intensité lumineuse de la lumière L ayant traversé la cuve 100. L'intensité de la lumière ayant traversé la cuve 100 emplie du milieu de culture comprenant la dispersion de microalgues est appelée intensité lumineuse de sortie $I_{out}$. Le capteur de lumière 300 mesure l'intensité lumineuse de sortie $I_{out}$ et transmet les données au contrôleur 400 pour le pilotage de la cuve. La transmission peut se faire directement ou indirectement via une commande 500 décrite ci-après.

Une commande 500 est reliée à la source de lumière 200 et permet de maintenir constante, d'augmenter ou de diminuer l'intensité lumineuse d'entrée $I_{in}$. La commande 500 est agencée pour régler l'intensité lumineuse d'entrée $I_{in}$ à une valeur consigne. Dans le mode de réalisation décrit ici, la commande 500 est également reliée au capteur de lumière 300. Le lien avec le capteur de lumière 300 permet à la commande 500 de recevoir des données relatives à l'intensité lumineuse de sortie $I_{out}$ mesurée par le capteur 300. La commande 500 peut ainsi transmettre ces données au contrôleur 400 pour que celui-ci puisse piloter la cuve en continu à la concentration consigne.

L'intensité lumineuse d'entrée $I_{in}$ et l'intensité lumineuse de sortie $I_{out}$ peuvent varier durant le temps de travail. Ainsi, la commande 500 est agencée pour régler l'intensité lumineuse d'entrée $I_{in}$ à la valeur consigne durant un temps inférieur ou égal au temps de travail.

La relation en fonction du temps entre l'intensité lumineuse d'entrée $I_{in}$ et l'intensité lumineuse de sortie $I_{out}$ est définie par la formule F2 suivante :

$$I_{out}(t) = I_{in}(t) \exp\left(-\sum_{i=1}^{n} a_i x_i P\right) \qquad \text{[F2]}$$

où $a_i$ est le coefficient d'atténuation des microalgues (c'est-à-dire la quantité de lumière absorbée par les microalgues) ; $x_i$ est la concentration des microalgues ; et P est la distance entre la source de lumière 200 et le capteur de lumière 300.

En régulant l'intensité lumineuse d'entrée $I_{in}$ la commande 500 peut également contrôler l'intensité lumineuse de sortie $I_{out}$.

Selon l'invention, la commande 500 est agencée pour régler l'intensité lumineuse d'entrée $I_{in}$ à une valeur de consigne durant un temps inférieur ou égale audit temps de travail pour induire un stress cellulaire chez certaines au moins des microalgues présentes dans la culture cellulaire. Dans un mode préférentiel, la commande 500 est agencée pour régler l'intensité lumineuse d'entrée $I_{in}$ à une valeur de consigne supérieure ou égale à 500 $\mu$mol quanta m$^{-2}$ s$^{-1}$.

La commande 500 est agencée pour recevoir des données relatives à la valeur de consigne. La valeur de consigne peut notamment varier en fonction d'une ou plusieurs espèces de micro-organismes photosynthétiques présentes dans la cuve 100. La valeur de consigne peut aussi varier en fonction de la concentration de culture cellulaire $x_i$ dans le milieu de culture.

La valeur de consigne peut évoluer durant le temps de travail. Dans un mode de réalisation, le temps de travail est composé d'une succession d'intervalles distincts de temps prédéfinis. Chaque intervalle est inférieur au temps de travail. Dans ce mode, la commande 500 peut régler l'intensité lumineuse d'entrée ($I_{in}$) à la valeur de consigne durant chacun desdits intervalles de temps. La valeur de consigne peut être fixée spécifiquement pour chaque intervalle. La valeur de consigne peut donc être différente d'un intervalle à l'autre. Dans un mode préférentiel, la valeur de consigne augmente d'un intervalle au suivant, par exemple par paliers de 100 à 200 $\mu$mol quanta m$^{-2}$ s$^{-1}$. Les intervalles peuvent être de même durée ou de durées différentes.

Dans un autre mode de réalisation, la commande 500 peut contrôler l'intensité lumineuse d'entrée $I_{in}$ en fonction de données relatives à l'intensité lumineuse de sortie $I_{out}$ mesurée par le capteur 300. Dans ce mode, la commande 500 peut être agencée pour recevoir des données du capteur 300, puis ajuster en fonction des données reçues l'intensité lumineuse d'entrée $I_{in}$ à la valeur consigne souhaitée.

La commande 500 peut être agencée pour maintenir l'intensité lumineuse d'entrée ($I_{in}$) à une valeur de consigne de sorte que l'intensité lumineuse en sortie ($I_{out}$) soit sensiblement constante durant un temps inférieur ou égale au temps de travail.

Dans un mode de réalisation, l'intensité lumineuse en sortie ($I_{out}$) peut être sensiblement constante durant la totalité du temps de travail. Dans ce mode, la commande 500 règle donc l'intensité lumineuse d'entrée ($I_{in}$) à la valeur de consigne et maintient celle-ci durant la totalité du temps de travail. L'intensité lumineuse ($I_{in}$) est, par exemple, ajustée en fonction de la concentration de de culture cellulaire $x_i$ dans le milieu de culture. Plus la concentration augmente, plus l'intensité lumineuse ($I_{in}$) augmente afin de maintenir l'intensité lumineuse en sortie ($I_{out}$) constante.

Dans un autre mode de réalisation, l'intensité lumineuse en sortie ($I_{out}$) est sensiblement constante durant un temps prédéfini inférieur au temps de travail. Dans ce mode, la commande 500 règle l'intensité lumineuse d'entrée ($I_{in}$)

à la valeur de consigne durant ce temps prédéfini (par exemple également en fonction de l'évolution de la concentration cellulaire dans le milieu).

Dans encore un autre mode de réalisation, l'intensité lumineuse en sortie ($I_{out}$) est sensiblement constante durant des intervalles de temps prédéfinis distincts, chaque intervalle de temps prédéfini étant inférieur au temps de travail. Dans ce mode, la commande 500 règle l'intensité lumineuse d'entrée ($I_{in}$) à la valeur de consigne durant chacun desdits intervalles de temps. La valeur de consigne peut être fixée spécifiquement pour chacun des intervalles. La valeur de consigne peut donc être différente d'un intervalle à l'autre. Dans un mode préférentiel, le temps de travail est composé d'une succession d'intervalles. La valeur de consigne évolue tout au long dudit temps de travail et augmente par paliers de 100 à 200 $\mu$mol quanta m$^{-2}$ s$^{-1}$ pour chaque intervalle. Les intervalles peuvent être de même durée ou de durées différentes.

Le fait d'induire un stress cellulaire auprès de certaines microalgues présente au moins deux conséquences :

- une première conséquence est que certaines microalgues touchées par le stress cellulaire peuvent être frappées du phénomène de photoinhibition. Certaines protéines clé de l'appareil photosynthétique sont endommagées et la croissance ralentie. Le phénomène peut aller jusqu'à l'arrêt du cycle cellulaire ; elles cessent de se diviser ou subissent la mort cellulaire ;
- une deuxième conséquence est que d'autres microalgues touchées par le stress cellulaire peuvent s'adapter au niveau génétique (notamment par mutations ponctuelles de nucléotides). Elles sont moins endommagées, et le cycle cellulaire de ces microalgues n'est pas stoppé ; elles présentent une résistance accrue à la photoinhibition. Comme expliqué plus haut, cette résistance est liée au fait que ces microalgues deviennent plus transparentes (teneur faible en pigments).

Le contrôleur 400 pilote la cuve 100 en continu à un taux de dilution D durant le temps de travail bien supérieur au temps nécessaire pour un cycle cellulaire de microalgues.

En conséquence, la cuve 100 se vide progressivement des cellules de microalgues touchées par la première conséquence. En effet, les cellules touchées cessent de se diviser, et leur croissance spécifique devient alors inférieure au débit ($\mu(x) < D$).

Par contre, les cellules de microalgues touchées par la deuxième conséquence restent représentées tout au long du temps de travail dans la cuve 100 car leur croissance spécifique ($\mu(x)$) n'est pas, ou peu, affectée.

D'autre part, les cellules des souches de microalgues présentant une résistance accrue à la photoinhibition et qui étaient déjà présentes dans la culture cellulaire restent également représentées dans la cuve 100 tout au long du temps de travail. Leur croissance spécifique ($\mu(x)$) n'est pas, ou peu, affectée.

De préférence, le temps de travail est supérieur au temps nécessaire pour au moins 20 cycles, de préférence 100 cycles, cellulaires de chaque cellule initiale de microalgue présente dans le milieu de culture. Dans le présent mode de réalisation le temps de travail est de 20 jours, et de préférence de 100 jours.

Dans un mode de réalisation, la commande 500 est agencée pour régler la lumière à une intensité lumineuse d'entrée à une valeur de repos $I_{in0}$. Ainsi, la commande 500 commute successivement entre une lumière d'intensité réglée à la valeur de repos et une lumière d'intensité réglée à la valeur de consigne.

Dans un mode de réalisation la valeur de repos est inférieure ou égale à 100 $\mu$mol quanta m$^{-2}$ s$^{-1}$. Ainsi, la commande 500 peut permuter successivement durant le temps de travail, entre la lumière incidente d'une intensité $I_{in0}$ inférieure ou égale à 100 $\mu$mol quanta m$^{-2}$ s$^{-1}$ (dite « intensité faible ») et une intensité $I_{in1}$ supérieure ou égale à 500 $\mu$mol quanta m$^{-2}$ s$^{-1}$ (dite « intensité forte »). Le temps de permutation T est inférieur au temps de travail (typiquement entre 30 minutes et 1 heure). Ainsi, la commande 500 peut commuter entre la lumière d'intensité lumineuse d'entrée $I_{in1}$ (dite « intensité forte »), pendant une fraction $p$ du temps, induisant un stress cellulaire et la lumière d'intensité faible $I_{in0}$, pendant une fraction (1-$p$) du temps. La fraction p est typiquement inférieure ou égale à 0.1. Ainsi, la commande 500 peut fixer un premier temps d'irradiation à intensité faible pour une durée $(1-p).T$ et un deuxième temps d'irradiation à intensité forte pour une durée $p.T$.

La lumière moyenne perçue par les cellules pour une culture à faible densité est alors

$$\bar{I}_{in} = p.I_{in1} + (1 - p).I_{in0}$$

Les paramètres ci-dessus sont choisis et/ou adaptés selon l'espèce des microalgues. Le choix est réalisé de sorte que d'une part, durant les phases d'intensité forte, le stress cellulaire de certaines au moins des microalgues présentes dans la culture cellulaire est induit, et que d'autre part, les cellules s'acclimatent à une lumière moyenne $\bar{I}_{in}$ faible, reflétant des conditions de culture en haute densité, elles seront ainsi en conditions de synthèse maximale de pigments.

Selon l'invention les temps d'irradiations sont fixés de sorte que la valeur moyenne de l'intensité lumineuse d'entrée ($I_{in}$) durant le temps de travail calculée à partir de la valeur de repos et la valeur de consigne soit inférieure ou égale à un tiers de ladite valeur de consigne. Dans mode préférentiel, le premier temps d'irradiation est au moins trois fois supérieur au deuxième temps d'irradiation. Une pluralité de premiers et deuxièmes temps d'irradiation peuvent être définis. Durant la pluralité de premiers et deuxièmes temps d'irradiation, la lumière peut être réglée à une intensité lumineuse d'entrée ($I_{in}$) à la valeur de repos lors de chaque premier temps d'irradiation et la lumière est réglée à une intensité lumineuse d'entrée ($I_{in}$) à la valeur consigne lors de chaque deuxième temps d'irradiation. La valeur de consigne peut être fixée pour chaque deuxième temps d'irradiation. De cette manière la valeur de consigne peut varier tout au long du temps de travail.

Dans un autre mode de réalisation la commande 500 règle l'intensité lumineuse d'entrée $I_{in}$ de sorte que l'intensité lumineuse en sortie $I_{out}$ soit sensiblement constante à une valeur suffisamment élevée pour induire de la photoinhibition. Par exemple cette valeur peut être supérieure ou égale à 500 $\mu$mol quanta m$^{-2}$ s$^{-1}$. Dans ce mode de réalisation, tout au long du temps de travail, les cellules de microalgues proches de la source de lumière 200 sont soumises à une intensité forte, c'est-à-dire soumises à une lumière d'intensité réglée à une valeur de consigne permettant d'induire un stress cellulaire auprès des microalgues. Au moins une partie des cellules de microalgues de la culture cellulaire est donc constamment soumise à un stress cellulaire. Ce mode de réalisation est particulièrement avantageux pour identifier des souches présentant une résistance accrue à la photoinhibition qui étaient initialement contenues dans la diversité initiale de la population de microalgues ou encore qui sont apparues par un processus de mutation génétique lors du temps de travail. Dans un mode de réalisation, le temps de travail est de 6 mois ; l'intensité lumineuse en sortie $I_{out}$ est de 1000 $\mu$mol quanta m$^{-2}$ s$^{-1}$; et le taux de dilution est de 0,3 j$^{-1}$.

Comme expliqué plus haut, dans ce mode de réalisation la valeur consigne peut évoluer par paliers au cours du temps de travail.

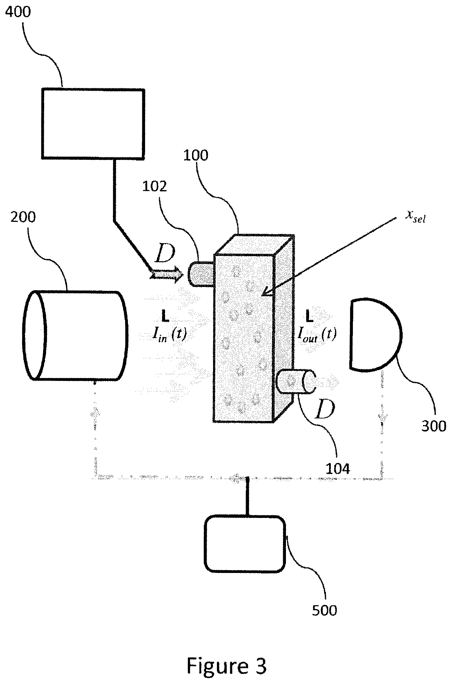

La figure 3 montre un schéma de principe du bioréacteur de la figure 2 de l'invention en fin de temps de travail.

Le milieu de culture dans la cuve 100 comprenait initialement une dispersion d'une culture cellulaire de microalgues comportant une variabilité naturelle d'individus plus ou moins résistantes à la photoinhibition de concentrations respectives $x_1$, $x_2$, $x_3$...$x_n$, les différentes souches de microalgues étant plus ou moins résistantes à la photoinhibition (cf. figure 2).

En fin du temps de travail, la cuve 100 comprend une dispersion d'une culture cellulaire de microalgues présentant une concentration $x_{sel}$ d'une ou plusieurs souches de microalgues présentant chacune une résistance accrue à la photoinhibition. Le contrôleur est agencé pour adapter le taux de dilution D durant le temps de travail. Dans un mode de réalisation, la concentration $x_{sel}$ est sensiblement identique à celle de la concentration $x_i$ initiale, à savoir comprise entre 0,05 g/L à 0,1 g/L. Le contrôleur 400 permet de fermer l'entrée 102 et la sortie 104 pour récolter la ou les souches de microalgues à résistance accrue à la photoinhibition. La récolte peut notamment être réalisée par prélèvement dans la cuve 100.

Le bioréacteur de l'invention permet donc d'obtenir des micro-organismes photosynthétiques à résistance accrue à la photo-inhibition. On entend par résistance accrue, une faible diminution du taux de croissance (de l'ordre de -25%) pour une intensité lumineuse deux fois supérieure à l'intensité optimale de croissance.

Plus l'intensité lumineuse est forte, plus la sélection est stricte. Avantageusement, le niveau de stress est augmenté progressivement durant le temps de travail. Ceci peut être réalisé en augmentant la moyenne de l'intensité lumineuse durant le temps de travail, notamment en augmentant l'intensité consigne au cours de l'avancement du temps de travail.

Le fonctionnement du bioréacteur de l'invention est indépendant du nombre de souches de microalgues présentes dans le milieu de culture. Dans d'autres modes de réalisation il peut s'agir d'une monoculture, à savoir une culture comportant qu'une seule souche de microalgues. Dans ces modes de réalisation, les cellules de microalgues peuvent être adaptées ou transformées par le bioréacteur de l'invention, par exemple par mutation ponctuelles au niveau de leurs séquences nucléotidiques.

**EXEMPLE**

L'exemple utilise un bioréacteur présentant une pluralité de sondes et capteurs. Le bioréacteur comprend notamment une sonde pour mesurer le pH et de température du milieu de culture, un capteur PAR pour mesurer le rayonnement photosynthétique, une sonde de turbidité pour mesurer la densité optique à 750 nm, un capteur fluorescent d'oxygène pour mesurer le taux de respiration et un capteur d'atténuation lumineuse réalisé par une photodiode captant la lumière $I_{in}$ ou une source additionnelle telle qu'une LED ou un faisceau laser.

Le bioréacteur présente également un capteur de niveau de type barrière laser à 600 nm. Le bioréacteur est couplé au Simulateur d'Environnement Marin Piloté par Ordinateur (SEMPO) permettant de reproduire expérimentalement l'environnement d'un milieu marin favorable pour la prolifération de microalgues, cf. The effects of a controlled fluctuating nutrient environment on continuous cultures of phytoplankton monitored by computers - Bernard, O., Malara, G., & Sciandra, A., Journal of Experimental Marine Biology and Ecology, 197(2), 263-278, 1996, et An automatic device for in vivo absorption spectra acquisition and chlorophyll estimation in phytoplankton cultures - Le Floc'h, E., Malara, G., & Sciandra, A., Journal of applied phycology, 14(6), 435-444, 2002. Le bioréacteur est également couplé à l'auto-analyseur TECHNICON qui permet de fixer des conditions de concentration en nutriments ($NO_2$ et $NO_3$). Le bioréacteur est aussi couplé à un compteur de particules de la société HIAC (marque déposée) permettant de suivre la production de biomasse.

Le pH du milieu de culture est régulé durant le temps de travail au moyen d'un générateur de rideau à bulles d'air et de $CO_2$ au sein du milieu de culture. La température du milieu de culture est régulée par un thermostat qui comprend la circulation d'eau à une température de consigne autour de la cuve du bioréacteur. La source de lumière comprend des rampes d'éclairage et/ou des filtres de lumière permettant d'éclairer selon différentes intensités lumineuses. L'apport en nutriments de la culture cellulaire est contrôlé par une dilution du milieu de culture, qui est fonction de la mesure de turbidité. Les nutriments nécessaires pour la production de biomasse peuvent être ajoutés en continu ou à fréquence variable.

La cuve est principalement réalisée en acier inoxydable d'un volume de 1.9 litres. La cuve présente deux façades en plexiglas (interchangeables avec des façades polycarbonate). Une première façade est disposée à proximité de la source de lumière de sorte que la lumière puisse pénétrer au sein de la cuve pour éclairer la culture cellulaire. Une deuxième façade est disposée à proximité du capteur de lumière de sorte que celui-ci puisse mesurer l'intensité lumineuse de sortie.

La source de lumière comprend une rampe d'éclairage à diodes électroluminescentes (LED) à haute puissance. La source de lumière est capable d'émettre une lumière d'intensité lumineuse de 5000 $\mu$mol photons m$^{-2}$ s$^{-1}$.

Le temps de travail est de 6 mois. La culture cellulaire consiste en une souche sauvage de cellules de *Tisochrysis lutea.*

Les cycles sont constitués de périodes d'environ 30 minutes à une intensité forte $I_{in1}$ (phases de stress) suivies de 90 minutes dans l'obscurité $I_{in0}$ (phase de repos). L'intensité lumineuse forte $I_{in1}$ est de 800 $\mu$mol photons m$^{-2}$ s$^{-1}$ au début du temps de travail, puis elle a été augmentée par palier au fil des cycles jusqu'à 1600 $\mu$mol photons m$^{-2}$ s$^{-1}$. Le tableau 1 ci-dessous montre le détail de chaque cycle :

Tableau 1 : paramètres des cycles de sélection appliqués. $I_{in1}$ : Lumière à une intensité réglée à la valeur consigne ; $I_{moyen}$ : Moyenne de l'intensité lumineuse sur un cycle ; $t_{in1}$ = Temps par créneau [c.à.d. Temps d'irradiation à forte intensité lumineuse (ou deuxième temps d'irradiation avec une intensité réglée à la valeur consigne)] ; $t_{in0}$ = Temps par créneau à $I_{in0}$ [c.à.d. Temps de repos ou temps d'irradiation à faible intensité lumineuse (ou premier temps d'irradiation avec une intensité réglée à la valeur de repos)].

| | $I_{in1}$ ($\mu$mol.m$^{-2}$.s$^{-1}$) | $I_{moyen}$ ($\mu$mol.m$^{-2}$.s$^{-1}$) | $I_{in1}$-$I_{moyen}$ ($\mu$mol.m$^{-2}$.s$^{-1}$) | $t_{in1}$ (seconde) | $t_{in0}$ (seconde) | Nombre de créneau (jour$^{-1}$) |
|---|---|---|---|---|---|---|
| **Cycle 1** | 800 | 200 | 600 | 1800 | 3600 | 16 |
| **Cycle 2** | 1000 | 300 | 700 | 2160 | 5040 | 12 |
| **Cycle 3** | 1200 | 350 | 850 | 2100 | 5100 | 12 |
| **Cycle 4** | 1400 | 350 | 1050 | 1800 | 5400 | 12 |
| **Cycle 5** | 1600 | 400 | 1200 | 1800 | 5400 | 12 |

Ici, la différence $I_{in1}$-$I_{moyen}$ est choisie de sorte à être au moins supérieur à 400 ($\mu$mol.m$^{-2}$.s$^{-1}$ ou de sorte que $I_{moyen}$ soit inférieur à un tiers de $I_{in1}$.

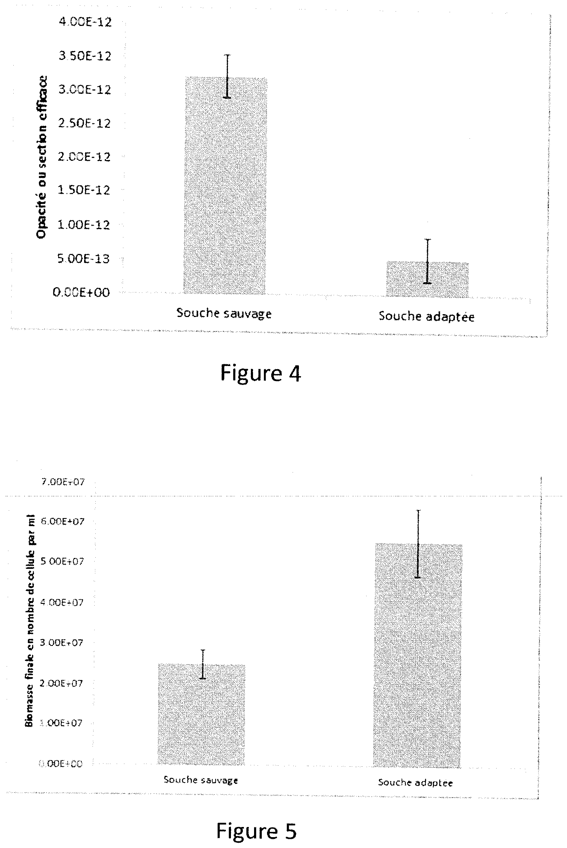

La figure 4 montre l'opacité de la souche sauvage de *Tisochrysis lutea* et l'opacité de la souche de *Tisochrysis lutea* sélectionnée par le bioréacteur de l'invention après le temps de travail. La souche sélectionnée présente une résistance accrue à la photoinhibition. Cette souche peut être qualifiée de « souche adaptée » car elle est issue de l'adaptation génétique (mutations ponctuelles) de la souche sauvage de *Tisochrysis lutea.*

Les mesures d'opacité (ou de section efficace) montrent que les cellules de la souche adaptée sont plus de six fois plus transparentes que la souche sauvage. La section efficace représente la surface totale d'antennes collectrices de lumière. Diminuer cette surface revient à rendre les microalgues plus transparentes.

La figure 5 montre la production de biomasse de la souche sauvage et la production de biomasse de la souche adaptée dans un système de culture classique de type *batch* en lumière continue avec une intensité lumineuse de 110 $\mu$mol photons m$^{-2}$ s$^{-1}$.

La production de biomasse de la souche adaptée est doublée par rapport à la production de biomasse de la souche sauvage. Le bioréacteur de l'invention présente donc un grand intérêt dans la production industrielle de biomasse de microalgues.

La biomasse est produite classiquement dans un photobioréacteur cylindrique en verre à une intensité lumineuse constante et de 110 $\mu$mol photons m$^{-2}$ s$^{-1}$, à température constante de 28°C et à un pH constant de 8.2, en un pilotage de type *batch.*

L'invention vise aussi une utilisation du bioréacteur tel que défini ci-dessus pour la production de biomasse d'une culture cellulaire choisie parmi le groupe constitué des classes Chlorophyceae telles que *Chlamydomonas sp., Dunaliella salina* et *Haematococcus pluvialis,* Bacillariophyceae (Diatomophyceae) telles que *Phaeodactylum tricornutum* et *Odontella aurita,* Isochrysidaceae telles que *Isochrysis galbana* et *Tisochrysis lutea,* Trebouxiophyceae telle que *Chlorella vulgaris,* et Cyanophyceae telle que *Arthrospira platensis.*

L'invention vise également un procédé de sélection de micro-organismes photosynthétiques tel que défini dans les revendications annexées.

Le procédé permet de sélectionner des souches de micro-organismes photosynthétiques présentant une résistance accrue à la photoinhibition parmi une population de micro-organismes photosynthétiques (ou culture cellulaire de micro-organismes photosynthétiques).

Les souches sélectionnées peuvent être utilisées pour la production de biomasse. L'exploitation industrielle des micro-organismes photosynthétiques sélectionnés offre de bons rendements en production. Le procédé comprend les étapes suivantes :

1. Mettre à disposition un bioréacteur comportant une source de lumière, et une cuve disposée en regard de la source de lumière destinée à recevoir un milieu de culture comprenant une culture cellulaire de micro-organismes photosynthétiques, ladite source de lumière étant capable d'émettre une lumière d'une intensité lumineuse d'entrée $I_{in}$ en direction de la cuve,

2. Emplir la cuve d'un milieu de culture ;

3. Inoculer le milieu de culture avec une culture cellulaire constituée de micro-organismes photosynthétiques ;

4. Piloter la cuve durant un temps de travail à une concentration de culture cellulaire $x_i$ choisie dans le milieu de culture ;

5. Régler l'intensité lumineuse d'entrée $I_{in}$, à une valeur de consigne permettant d'induire un stress cellulaire chez certains au moins desdits micro-organismes photosynthétiques ;

6. Maintenir l'intensité lumineuse d'entrée $I_{in}$, à la valeur de consigne durant un temps inférieur ou égal audit temps de travail ; de manière à provoquer une dégradation cellulaire d'au moins une partie desdits micro-organismes photosynthétiques et ainsi sélectionner les micro-organismes photosynthétiques présentant une résistance accrue à la photoinhibition ; et

7. Récolter les micro-organismes photosynthétiques présentant une résistance accrue à la photoinhibition dudit milieu de culture tel que défini dans les revendications annexées.

On entend par dégradation cellulaire toute dégradation ayant pour conséquence l'élimination d'une cellule du milieu de culture. Il peut tout particulièrement s'agir de la mort cellulaire. La mort cellulaire peut-être causée par des dommages physiques ou chimiques - il s'agit de la nécrose cellulaire ; mort cellulaire peut-être causée le suicide de la cellule - il s'agit de l'apoptose. De plus, il peut s'agir de la mort mitotique (arrêt de la division cellulaire) dans laquelle la cellule ne peut plus se reproduire et fini par être évacuée du bioréacteur lorsque celui-ci est piloté en continu.

La dégradation des cellules sensibles au stress cellulaire a pour conséquence que la population de cellules résistantes (ou devenues résistantes par mutation génétique) augmente dans le milieu de culture. Ainsi, la récolte à l'étape 7. est réalisée lorsque le milieu de culture comporte une culture cellulaire de micro-organismes photosynthétiques constituée à plus de 75%, préférentiellement à plus de 90%, encore plus préférentiellement sensiblement à 100% de micro-organismes présentant une résistance accrue à la photoinhibition.

Selon l'invention, l'étape 6 comprend comprendre les sous-étapes suivantes:

6a. Régler la lumière à une intensité lumineuse d'entrée $I_{in}$ à une valeur de repos inférieure à la valeur de consigne durant un temps inférieur audit temps de travail ;

6b. Maintenir l'intensité lumineuse d'entrée $I_{in}$, à la valeur de repos durant un temps inférieur audit temps de travail ; et

6c. Commuter successivement entre la valeur de repos et la valeur de consigne durant le temps de travail.

Bien évidemment, dans ce mode de réalisation le maintien à l'étape 6. de l'intensité lumineuse d'entrée $I_{in}$, à la valeur de consigne s'effectue durant un temps inférieur au temps de travail. Ceci est nécessaire pour pouvoir commuter entre la valeur de repos et la valeur de consigne tout au long du temps de travail.

Selon l'invention, le procédé comprend les sous-étapes suivantes :

- fixer un premier temps d'irradiation pour lequel la lumière est réglée à l'intensité lumineuse d'entrée $I_{in}$ à la valeur de repos ; et
- fixer un deuxième temps d'irradiation pour lequel la lumière est réglée à la valeur de consigne.

Pour des raisons de photo-acclimatation, le premier temps d'irradiation est de préférence supérieur au deuxième temps d'irradiation. Ce mode de réalisation résulte donc en la succession de premiers temps d'irradiations et de deuxième temps d'irradiations. Chaque premier temps d'irradiation durant lequel la lumière est réglée à l'intensité lumineuse d'entrée $I_{in}$ à la valeur de repos, est suivi d'un deuxième temps d'irradiation, durant lequel la lumière est réglée est réglée à l'intensité lumineuse d'entrée $I_{in}$ à la valeur de consigne. Les cellules de microalgues sont ainsi soumises à la succession de phases de stress et de phases de photo-acclimatation (ou phases de repos). Il faut noter que l'intensité lumineuse d'entrée $I_{in}$ à la valeur de repos peut correspondre en l'absence de lumière, à savoir à l'obscurité ; donc 0 $\mu$mol quanta m$^{-2}$ s$^{-1}$. Dans d'autres mode de réalisation la valeur de repose peut être comprise entre 10 et 200 $\mu$mol quanta m$^{-2}$ s$^{-1}$, par exemple 50 $\mu$mol quanta m$^{-2}$ s$^{-1}$ ou 100 $\mu$mol quanta m$^{-2}$ s$^{-1}$.

Selon l'invention, les premiers et deuxième temps d'irradiations sont fixés de sorte que la valeur moyenne de l'intensité lumineuse d'entrée $I_{in}$ durant le temps de travail calculée à partir de la valeur de repos et la valeur de consigne soit inférieure ou égale à un tiers de ladite valeur de consigne. Cela assure un temps de repos suffisant pour que les cellules puissent s'acclimater aux conditions de stress. Dans un mode particulier de réalisation, le premier temps d'irradiation est au moins trois fois supérieur au deuxième temps d'irradiation.

De préférence, le temps de travail est supérieur au temps nécessaire pour au moins 20 cycles, de préférence 100 cycles, cellulaires de chaque cellule initiale de microalgue présente dans le milieu de culture.

Avantageusement, le procédé met en oeuvre une répétition successive des étapes 5. et 6. jusqu'à l'obtention d'une culture cellulaire de micro-organismes photosynthétiques sensiblement constituée de micro-organismes présentant une résistance accrue à la photoinhibition. La répétition des ces étapes permet une sélection satisfaisante et favorise la photo-acclimatation des cellules.

Le procédé peut en outre comprendre les étapes suivantes :

8. Inoculer un milieu de culture avec les micro-organismes photosynthétiques récoltés à l'étape 7. dans un bioréacteur ; et
9. Piloter le bioréacteur de l'étape 8. pour une production de biomasse constituée desdits micro-organismes photosynthétiques.

Cette étape peut-être réalisée dans le même bioréacteur ou dans un bioréacteur distinct particulièrement adapté pour la production de biomasse. Par exemple, le bioréacteur peut-être un bassin de culture (bassin type « raceway») et un système de culture fermé comme les bioréacteurs d'alimentation par *batch,* les bioréacteurs d'alimentation par *fed-batch* ou les bioréacteurs d'alimentation continu.

Le procédé de l'invention peut en outre comprendre une sous-étape 4a. qui comporte la mesure de l'intensité lumineuse en sortie $I_{out}$. L'intensité lumineuse d'entrée $I_{in}$ à l'étape 5. peut alors être réglée en fonction de l'intensité lumineuse en sortie $I_{out}$ mesurée à l'étape 4a. Dans ce mode de réalisation, le bioréacteur comprend un capteur de lumière disposé à l'opposé de ladite source de lumière par rapport à la cuve (le capteur est en regard de la source de lumière). Le capteur de lumière est agencé de manière à mesurer l'intensité lumineuse de sortie ($I_{out}$).

De préférence, la valeur de consigne de l'intensité lumineuse d'entrée $I_{in}$ est supérieure ou égale à 500 $\mu$mol quanta m$^{-2}$ s$^{-1}$. Dans un mode particulier de réalisation, la valeur de consigne est supérieure ou égale à 800 $\mu$mol quanta m$^{-2}$ s$^{-1}$.

Dans un autre mode de réalisation particulier, la valeur de consigne augmente par paliers au cours du temps de travail (par exemple par paliers de 200 $\mu$mol quanta m$^{-2}$ s$^{-1}$). Ceci permet d'augmenter davantage la sélection. On abouti alors à une sélection plus stricte. En fin du temps de travail, seules les microalgues hautement résistantes à la photoinhibition subsistes dans le milieu de culture. Les valeurs de consignes suivantes peuvent notamment être appliquées de manière successive : 800 $\mu$mol quanta m$^{-2}$ s$^{-1}$ ; 1000 $\mu$mol quanta m$^{-2}$ s$^{-1}$ ; 1200 $\mu$mol quanta m$^{-2}$ s$^{-1}$ ; 1400 $\mu$mol quanta m$^{-2}$ s$^{-1}$ ; 1600 $\mu$mol quanta m$^{-2}$ s$^{-1}$. Les phases durant lesquels l'intensité lumineuse d'entrée $I_{in}$ est réglée aux valeurs de consignes, peuvent être optionnellement suivies de phases durant lesquels l'intensité lumineuse d'entrée $I_{in}$ est réglée à la valeur de repos, par exemple à une valeur proche ou égale à 0 $\mu$mol quanta m$^{-2}$ s$^{-1}$ (obscurité) ou proche de 50 à 100 $\mu$mol quanta m$^{-2}$ s$^{-1}$. Cela permet aux microalgues de s'acclimater et le cas échéant d'engager une modification au niveau moléculaire (mutations génétiques notamment).

De préférence, le pilotage à l'étape 4. s'effectue à une concentration (par exemple à une valeur comprise 0,001 g/L à 10 g/L, de préférence entre 0,05 g/L à 0,1 g/L ou entre 0,1 g/L à 5 g/L).

## Revendications

1. Bioréacteur **caractérisé en ce qu'**il comporte une source de lumière (200), un capteur de lumière (300) en regard de ladite source de lumière et une cuve (100) disposée entre la source de lumière (200) et le capteur de lumière (300) destinée à recevoir un milieu de culture comprenant une culture cellulaire de micro-organismes photosynthétiques, un contrôleur (400) relié au capteur de lumière (300) pour piloter la cuve (100) à une concentration de culture cellulaire choisie ($x_i$) dans le milieu de culture durant un temps de travail, ladite source de lumière (200) étant capable d'émettre une lumière incidente (L) d'une intensité lumineuse d'entrée ($I_{in}$) en direction de la cuve (100), et le capteur de lumière (300) étant capable de mesurer une intensité lumineuse en sortie ($I_{out}$) et de transmettre des données relatives à cette intensité ($I_{out}$) au contrôleur pour le pilotage de la cuve, et une commande (500) de la source de lumière (200) agencée pour régler, durant un temps inférieur audit temps de travail, l'intensité lumineuse d'entrée ($I_{in}$) à une valeur de consigne permettant d'induire un stress cellulaire chez certains au moins desdits micro-organismes photosynthétiques et dans lequel la commande est en outre agencée pour régler ladite lumière à une intensité lumineuse d'entrée ($I_{in}$) à une valeur de repos inférieure à la valeur de consigne, et pour commuter entre la valeur de repos et la valeur de consigne durant le temps de travail, et dans lequel la commande est en outre agencée pour fixer un premier temps d'irradiation pour lequel la lumière est réglée à l'intensité lumineuse d'entrée ($I_{in}$) à la valeur de repos et un deuxième temps d'irradiation pour lequel la lumière est réglée à la valeur de consigne, le premier temps d'irradiation étant supérieur au deuxième temps d'irradiation, et pour appliquer une succession des premiers et deuxième temps d'irradiations, et dans lequel les premiers et deuxième temps d'irradiations sont fixés de sorte que la valeur moyenne de l'intensité lumineuse d'entrée ($I_{in}$) durant le temps de travail calculée à partir de la valeur de repos et la valeur de consigne soit inférieure ou égale à un tiers de ladite valeur de consigne.

2. Bioréacteur selon la revendication 1, dans lequel la valeur de consigne est supérieure ou égale à 500 $\mu$mol quanta m$^{-2}$ s$^{-1}$, de préférence supérieure ou égale à 800 $\mu$mol quanta m$^{-2}$ s$^{-1}$.

3. Bioréacteur selon l'une des revendications précédentes, dans lequel la concentration de culture cellulaire choisie ($x_i$) dans le milieu de culture est comprise entre 0,001 g/l à 0,1 g/L, de préférence entre 0,05 g/l à 0,1 g/L.

4. Bioréacteur selon l'une des revendications précédentes, dans lequel le premier temps d'irradiation est au moins trois fois supérieur au deuxième temps d'irradiation.

5. Bioréacteur selon l'une des revendications précédentes, dans lequel la commande est en outre agencée pour fixer une pluralité de premiers et deuxièmes temps d'irradiation durant lesquels la lumière est réglée à une intensité lumineuse d'entrée ($I_{in}$) à la valeur de repos lors de chaque premier temps d'irradiation et la lumière est réglée à une intensité lumineuse d'entrée ($I_{in}$) à la valeur consigne lors de chaque deuxième temps d'irradiation.

6. Bioréacteur selon la revendication 5, dans lequel la commande est en outre agencée pour fixer la valeur de consigne à chaque deuxième temps d'irradiation.

7. Bioréacteur selon l'une des revendications 1 et 2, dans lequel la concentration de culture cellulaire choisie ($x_i$) dans le milieu de culture est comprise entre 0,1 g/l à 10 g/L, de préférence entre 1 g/l à 10 g/L.

8. Bioréacteur selon la revendication 7, dans lequel la commande est agencée pour maintenir l'intensité lumineuse d'entrée ($I_{in}$) à la valeur de consigne de sorte que l'intensité lumineuse en sortie ($I_{out}$) soit sensiblement constante durant un temps inférieur ou égale au temps de travail.

9. Procédé de sélection de micro-organismes photosynthétiques, comprenant les étapes suivantes :

1. Mettre à disposition un bioréacteur comportant une source de lumière, et une cuve disposée en regard de la source de lumière destinée à recevoir un milieu de culture comprenant une culture cellulaire de micro-organismes photosynthétiques, ladite source de lumière étant capable d'émettre une lumière d'une intensité lumineuse d'entrée $I_{in}$ en direction de la cuve,
2. Emplir la cuve d'un milieu de culture ;
3. Inoculer le milieu de culture avec une culture cellulaire constituée de micro-organismes photosynthétiques ;

4. Piloter la cuve durant un temps de travail à une concentration de culture cellulaire $x_i$ choisie dans le milieu de culture ;

5. Régler l'intensité lumineuse d'entrée $I_{in}$, à une valeur de consigne permettant d'induire un stress cellulaire chez certains au moins desdits micro-organismes photosynthétiques ;

6. Maintenir l'intensité lumineuse d'entrée $I_{in}$, à la valeur de consigne durant un temps inférieur ou égal audit temps de travail de manière à provoquer une dégradation cellulaire d'au moins une partie desdits micro-organismes photosynthétiques et ainsi sélectionner les micro-organismes photosynthétiques présentant une résistance accrue à la photoinhibition ; et

7. Récolter les micro-organismes photosynthétiques présentant une résistance accrue à la photoinhibition dudit milieu de culture

dans lequel l'étape 6 comporte les sous-étapes suivantes :

6a. Régler la lumière à une intensité lumineuse d'entrée à une valeur de repos inférieure à la valeur de consigne durant un temps inférieur audit temps de travail ;

6b. Maintenir l'intensité lumineuse d'entrée à la valeur de repos durant un temps inférieur audit temps de travail ; et

6c. Commuter successivement entre la valeur de repos et la valeur de consigne durant le temps de travail ;

et dans lequel le procédé comporte en outre les sous-étapes de :

- fixer un premier temps d'irradiation pour lequel la lumière est réglée à l'intensité lumineuse d'entrée à la valeur de repos ; et de
- fixer un deuxième temps d'irradiation pour lequel la lumière est réglée à la valeur de consigne,

les premiers et deuxième temps d'irradiations étant fixés de sorte que la valeur moyenne de l'intensité lumineuse d'entrée $I_{in}$ durant le temps de travail calculée à partir de la valeur de repos et la valeur de consigne soit inférieure ou égale à un tiers de ladite valeur de consigne.

10. Procédé selon la revendication 9, dans lequel la récolte à l'étape 7. est réalisée lorsque le milieu de culture comporte une culture cellulaire de micro-organismes photosynthétiques constituée à plus de 75%, préférentiellement à plus de 90%, encore plus préférentiellement sensiblement à 100% de micro-organismes présentant une résistance accrue à la photoinhibition.

11. Procédé selon l'une des revendications 9 et 10, comprenant en outre les étapes suivantes :

8. Inoculer un milieu de culture avec les micro-organismes photosynthétiques récoltés à l'étape 7. dans un bioréacteur ; et

9. Piloter le bioréacteur de l'étape 8. pour une production de biomasse constituée desdits micro-organismes photosynthétiques.

12. Procédé selon l'une des revendications 9 à 10, dans lequel le bioréacteur comprend en outre un capteur de lumière à l'opposé de ladite source de lumière par rapport à la cuve, le capteur de lumière étant capable de mesurer une intensité lumineuse de sortie ($I_{out}$) ; le procédé comprenant en outre l'étape 4a. de mesurer l'intensité lumineuse en sortie ($I_{out}$), et dans lequel l'intensité lumineuse d'entrée ($I_{in}$) à l'étape 5. est réglée en fonction de l'intensité lumineuse en sortie ($I_{out}$) mesurée à l'étape 4a.

## Patentansprüche

1. Bioreaktor, **dadurch gekennzeichnet, dass** er eine Lichtquelle (200), einen Lichtsensor (300) gegenüber der Lichtquelle und einen zwischen der Lichtquelle (200) und dem Lichtsensor (300) angeordneten Tank (100), der dazu vorgesehen ist, ein Kulturmedium aufzunehmen, das eine Zellkultur von photosynthetischen Mikroorganismen umfasst, eine mit dem Lichtsensor (300) verbundene Steuereinheit (400) zum Steuern des Tanks (100) auf eine gewählte Zellkultur-Konzentration ($x_i$) im Kulturmedium während einer Betriebszeit, wobei die Lichtquelle (200) dazu fähig ist, ein einfallendes Licht (L) mit einer Eingangslichtstärke ($I_{in}$) in Richtung des Tanks (100) auszustrahlen, und der Lichtsensor (300) dazu fähig ist, eine Ausgangslichtstärke ($I_{out}$) zu messen und Daten im Zusammenhang mit dieser Intensität ($I_{out}$) zur Steuereinheit zum Steuern des Tanks zu übertragen, und eine Steuerung (500) der Lichtquelle (200) umfasst, die dazu eingerichtet ist, während einer Zeit, die kleiner als die Betriebszeit ist, die Eingangslichtstärke

($I_{in}$) auf einen Einstellwert zu regeln, der es ermöglicht, bei zumindest einigen der photosynthetischen Mikroorganismen einen Zellstress zu induzieren, und wobei die Steuerung weiterhin dazu eingerichtet ist, das Licht auf eine Eingangslichtstärke ($I_{in}$) bei einem Ruhewert zu regeln, der niedriger als der Einstellwert ist, und um während der Betriebszeit zwischen dem Ruhewert und dem Einstellwert umzuschalten, und wobei die Steuerung weiterhin dazu eingerichtet ist, eine erste Bestrahlungszeit, in der das Licht auf die Eingangslichtstärke ($I_{in}$) beim Ruhewert geregelt wird, und eine zweite Bestrahlungszeit festzulegen, in der das Licht auf den Einstellwert geregelt wird, wobei die erste Bestrahlungszeit größer als die zweite Bestrahlungszeit ist, und um eine Abfolge von ersten und zweiten Bestrahlungszeiten anzuwenden, und wobei die ersten und die zweiten Bestrahlungszeiten so festgelegt sind, dass der Mittelwert der Eingangslichtstärke ($I_{in}$) während der Betriebszeit, der aus dem Ruhewert und dem Einstellwert berechnet wird, kleiner als ein oder gleich einem Drittel des Einstellwerts ist.

2. Bioreaktor nach Anspruch 1, wobei der Einstellwert größer als oder gleich 500 $\mu$mol Quanten m$^{-2}$ s$^{-1}$, vorzugsweise größer als oder gleich 800 $\mu$mol Quanten m$^{-2}$ s$^{-1}$, ist.

3. Bioreaktor nach einem der vorhergehenden Ansprüche, wobei die gewählte Zellkultur-Konzentration ($x_i$) im Kulturmedium zwischen 0,001 g/l und 0,1 g/l, vorzugsweise zwischen 0,05 g/l und 0,1 g/l, beträgt.

4. Bioreaktor nach einem der vorhergehenden Ansprüche, wobei die erste Bestrahlungszeit mindestens drei Mal größer als die zweite Bestrahlungszeit ist.

5. Bioreaktor nach einem der vorhergehenden Ansprüche, wobei die Steuerung weiterhin dazu eingerichtet ist, mehrere erste und zweite Bestrahlungszeiten festzulegen, in denen das Licht bei jeder ersten Bestrahlungszeit auf eine Eingangslichtstärke ($I_{in}$) beim Ruhewert geregelt wird und das Licht bei jeder zweiten Bestrahlungszeit auf eine Eingangslichtstärke ($I_{in}$) beim Einstellwert geregelt wird.

6. Bioreaktor nach Anspruch 5, wobei die Steuerung weiterhin dazu eingerichtet ist, den Einstellwert bei jeder zweiten Bestrahlungszeit festzulegen.

7. Bioreaktor nach einem der Ansprüche 1 und 2, wobei die gewählte Zellkultur-Konzentration ($x_i$) im Kulturmedium zwischen 0,1 g/l und 10 g/l, vorzugsweise zwischen 1 g/l und 10 g/l, beträgt.

8. Bioreaktor nach Anspruch 7, wobei die Steuerung dazu eingerichtet ist, die Eingangslichtstärke ($I_{in}$) beim Einstellwert so zu halten, dass die Ausgangslichtstärke ($I_{out}$) während einer Zeit, die kleiner als die oder gleich der Betriebszeit ist, im Wesentlichen konstant ist.

9. Verfahren zur Auswahl von photosynthetischen Mikroorganismen, das die folgenden Schritte umfasst:

1. das Bereitstellen eines Bioreaktors, der eine Lichtquelle und einen gegenüber der Lichtquelle angeordneten Tank umfasst, der dazu vorgesehen ist, ein Kulturmedium aufzunehmen, das eine Zellkultur von photosynthetischen Mikroorganismen umfasst, wobei die Lichtquelle dazu fähig ist, ein Licht mit einer Eingangslichtstärke $I_{in}$ in Richtung des Tanks auszustrahlen,
2. das Füllen des Tanks mit einem Kulturmedium;
3. das Impfen des Kulturmediums mit einer Zellkultur, die aus photosynthetischen Mikroorganismen besteht;
4. das Steuern des Tanks bei einer gewählten Zellkultur-Konzentration $x_i$ im Kulturmedium für eine Betriebszeit;
5. das Regeln der Eingangslichtstärke $I_{in}$ auf einen Einstellwert, der es ermöglicht, bei zumindest einigen der photosynthetischen Mikroorganismen einen Zellstress zu induzieren;
6. das Halten der Eingangslichtstärke $I_{in}$ während einer Zeit, die kleiner als die oder gleich der Betriebszeit ist, auf dem Einstellwert, um einen Zellabbau zumindest eines Teils der photosynthetischen Mikroorganismen hervorzurufen und so die photosynthetischen Mikroorganismen auszuwählen, die eine erhöhte Beständigkeit gegenüber der Photoinhibition aufweisen; und
7. das Ernten der photosynthetischen Mikroorganismen, die eine erhöhte Beständigkeit gegenüber der Photoinhibition aufweisen, aus dem Kulturmedium,
wobei Schritt 6 die folgenden Teilschritte umfasst:

6a. das Regeln des Lichts während einer Zeit, die kleiner als die Betriebszeit ist, auf eine Eingangslichtstärke bei einem Ruhewert, der kleiner als der Einstellwert ist;
6b. das Halten der Eingangslichtstärke während einer Zeit, die kleiner als die Betriebszeit ist, auf dem Ruhewert; und

6c. während der Betriebszeit nacheinander das Umschalten zwischen dem Ruhewert und dem Einstellwert;

und wobei das Verfahren weiterhin die folgenden Teilschritte umfasst:

- das Festlegen einer ersten Bestrahlungszeit, in der das Licht auf eine Eingangslichtstärke beim Ruhewert geregelt wird; und
- das Festlegen einer zweiten Bestrahlungszeit, in der das Licht auf den Einstellwert geregelt wird,

wobei die erste und die zweite Bestrahlungszeit so festgelegt sind, dass der Mittelwert der Eingangslichtstärke $I_{in}$ während der Betriebszeit, der aus dem Ruhewert und dem Einstellwert berechnet wird, kleiner als ein oder gleich einem Drittel des Einstellwerts ist.

**10.** Verfahren nach Anspruch 9, wobei die Ernte in Schritt 7. erfolgt, wenn das Kulturmedium eine Zellkultur von photosynthetischen Mikroorganismen umfasst, die zu mehr als 75 %, vorzugsweise zu mehr als 90 %, noch mehr bevorzugt im Wesentlichen zu 100 % aus Mikroorganismen besteht, die eine erhöhte Beständigkeit gegenüber der Photoinhibition aufweisen.

**11.** Verfahren nach einem der Ansprüche 9 und 10, das weiterhin die folgenden Schritte umfasst:

8. das Impfen eines Kulturmediums mit den in Schritt 7. geernteten photosynthetischen Mikroorganismen in einem Bioreaktor und
9. das Steuern des Bioreaktors von Schritt 8. für eine Produktion von Biomasse, die aus den photosynthetischen Mikroorganismen besteht.

**12.** Verfahren nach einem der Ansprüche 9 bis 10, wobei der Bioreaktor weiterhin einen Lichtsensor gegenüber der Lichtquelle in Bezug auf den Tank umfasst, der Lichtsensor dazu fähig ist, eine Ausgangslichtstärke ($I_{out}$) zu messen; wobei das Verfahren weiterhin den Schritt 4a. des Messens der Ausgangslichtstärke ($I_{out}$) umfasst und wobei die Eingangslichtstärke ($I_{in}$) in Schritt 5. in Abhängigkeit von der in Schritt 4a. gemessenen Ausgangslichtstärke ($I_{out}$) geregelt wird.

**Claims**

**1.** A bioreactor **characterized in that** it comprises a light source (200), a light sensor (300) facing said light source and a tank (100) arranged between the light source (200) and the light sensor (300) intended to receive a culture medium comprising a cell culture of photosynthetic microorganisms, a controller (400) connected to the light sensor (300) to control the tank (100) to a chosen cell culture concentration ($x_i$) in the culture medium during a working period, said light source (200) being capable of emitting an incident light (L) with an input light intensity ($I_{in}$) in the direction of the tank (100), and the light sensor (300) being capable of measuring an output light intensity ($I_{out}$) and of transmitting data relating to this intensity ($I_{out}$) to the controller for controlling the tank, and a system (500) for controlling the light source (200), arranged to set, for a period less than or equal to said working period, the input light intensity ($I_{in}$) to a setpoint value enabling to induce cellular stress in at least some of said photosynthetic microorganisms, and wherein the system is also arranged to set said light to an input light intensity ($I_{in}$) at a resting value lower than the setpoint value, and to switch between the resting value and the setpoint value during the working period, and wherein the system is also arranged to fix a first irradiation period for which the light is set to the input light intensity ($I_{in}$) at the resting value and a second irradiation period for which the light is set to the setpoint value, the first irradiation period being greater than the second irradiation period, and to apply a succession of the first and second irradiation periods; and wherein the first and second irradiation periods are fixed such that the mean value of the input light intensity ($I_{in}$) during the working period, calculated from the resting value and the setpoint value, is less than or equal to a third of said setpoint value.

**2.** The bioreactor as claimed in claim 1, wherein the setpoint value is greater than or equal to 500 $\mu$mol quanta m$^{-2}$ s$^{-1}$, preferably greater than or equal to 800 $\mu$mol quanta m$^{-2}$ s$^{-1}$.

**3.** The bioreactor according to one of the preceding claims, wherein the chosen cell culture concentration ($x_i$) in the culture medium is between 0.001 g/l and 0.1 g/L, preferably between 0.05 g/l and 0.1 g/L.

**4.** The bioreactor according to one of the preceding claims, wherein the first irradiation period is at least three times greater than the second irradiation period.

5. The bioreactor according to one of the preceding claims, wherein the control system is also arranged to fix a plurality of first and second irradiation periods during which the light is set to an input light intensity ($I_{in}$) at the resting value during each first irradiation period and the light is set to an input light intensity ($I_{in}$) at the setpoint value during each second irradiation period.

6. The bioreactor as claimed in claim 5, wherein the control system is also arranged to fix the setpoint value at each second irradiation period.

7. The bioreactor as claimed in one of claims 1 and 2, wherein the chosen cell culture concentration ($x_i$) in the culture medium is between 0.1 g/l and 10 g/L, preferably between 1 g/l and 10 g/L.

8. The bioreactor as claimed in claim 7, wherein the control system is arranged to maintain the input light intensity ($I_{in}$) at the setpoint value, such that the output light intensity ($I_{out}$) is substantially constant for a period less than or equal to the working period.

9. A process for selecting photosynthetic microorganisms, comprising the following steps:

   1. Providing a bioreactor comprising a light source, and a tank arranged facing the light source intended to receive a culture medium comprising a cell culture of photosynthetic microorganisms, said light source being capable of emitting a light with an input light intensity $I_{in}$ in the direction of the tank,
   2. Filling the tank with a culture medium;
   3. Inoculating the culture medium with a cell culture composed of photosynthetic microorganisms;
   4. Controlling the tank for a working period to a chosen cell culture concentration $x_i$ in the culture medium;
   5. Setting the input light intensity $I_{in}$ to a setpoint value enabling to induce cellular stress in at least some of said photosynthetic microorganisms;
   6. Maintaining the input light intensity $I_{in}$ at the setpoint value for a period less than or equal to said working period, so as to cause cellular degradation of at least a portion of said photosynthetic microorganisms and thereby select the photosynthetic microorganisms having increased resistance to photoinhibition, and
   7. Harvesting the photosynthetic microorganisms having increased resistance to photoinhibition from said culture medium.
   wherein step 6 comprises the following steps:

      6a. Setting the light to an input light intensity, at a resting value lower than the setpoint value, for a period less than said working period;
      6b. Maintaining the input light intensity at the resting value for a period less than said working period; and
      6c. Successively switching between the resting value and setpoint value during the working period;

   and wherein the process further comprises the steps of:

      - fixing a first irradiation period for which the light is set to an input light intensity at the resting value; and
      - fixing a second irradiation period for which the light is set to the setpoint value,

   the first and second irradiation periods are fixed such that the mean value of the input light intensity during the working period, calculated from the resting value and the setpoint value, is less than or equal to a third of said setpoint value.

10. The process as claimed in claim 9, wherein the harvesting at step 7. is carried out when the culture medium comprises a cell culture of photosynthetic microorganisms consisting of more than 75%, preferentially of more than 90%, even more preferentially substantially of 100%, of microorganisms having increased resistance to photoinhibition.

11. The process as claimed in one of claims 9 and 10, also comprising the following steps:

   8. Inoculating a culture medium with the photosynthetic microorganisms harvested in step 7. in a bioreactor; and
   9. Controlling the bioreactor of step 8. for production of biomass formed of said photosynthetic microorganisms.

12. The process as claimed in one of claims 9 to 10, wherein the bioreactor also comprises a light sensor opposite said light source relative to the tank, the light sensor being capable of measuring an output light intensity ($I_{out}$); the process also comprising the step 4a. of measuring the output light intensity ($I_{out}$), and wherein the input light intensity ($I_{in}$) at step 5. is set as a function of the output light intensity ($I_{out}$) measured in step 4a.

Figure 1

Figure 2

Figure 3

Figure 4

Figure 5

**EP 3 440 185 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014089533 A
- WO 2013012329 A
- US 20070231886 A
- US 5882849 A
- US 20140356902 A
- KR 1020040059182

**Littérature non-brevet citée dans la description**

- **CARVALHO et al.** Light requirements in microalgal photobioreactors: on overview of biophotonic aspects. *Appl Microbiology and Biotechnology,* 2011, vol. 89 (5), 1275-1288
- **SCHULZE et al.** Light emitting diodes (LEDs) applied to microalgal production. *Trends in Biotechnology,* 2014, vol. 32 (8), 422-430
- **MÜLLER et al.** Non-Photochemical Quenching. A Response to Excess Light Energy. *Plant Physiol,* 2001, vol. 125 (4), 1558-1566
- **C. POSTEN ; C. WALTER.** Microalgal biotechnology: potential and production. de Gruyter, 2012
- **A. RICHMOND ; Q. HU.** Handbook of Microalgal Culture: Applied Phycology and Biotechnology. Wiley-Blackwell, 2013
- **BASHAN, L. E. ; TREJO, A. ; HUSS, V. A. ; HERNANDEZ, J. P. ; BASHAN, Y.** Chlorella sorokiniana UTEX 2805, a heat and intense, sunlight-tolérant microalga with potential for removing ammonium from wastewater. *Bioresource Technology,* 2008, vol. 99 (11), 4980-4989
- **BERNARD, O. ; MALARA, G. ; SCIANDRA, A.** The effects of a controlled fluctuating nutrient environment on continuous cultures of phytoplankton monitored by computers. *Journal of Experimental Marine Biology and Ecology,* 1996, vol. 197 (2), 263-278
- **LE FLOC'H, E. ; MALARA, G. ; SCIANDRA, A.** An automatic device for in vivo absorption spectra acquisition and chlorophyll estimation in phytoplankton cultures. *Journal of applied phycology,* 2002, vol. 14 (6), 435-444